# EUROPEAN PATENT SPECIFICATION

(11) **EP 0 537 278 B1**
(45) Date of publication and mention of the grant of the patent: **11.09.1996**
(21) Application number: 91913507.9
(22) Date of filing: 03.07.1991
(51) Int. Cl.: A61M 29/02, A61M 25/01

(54) **PTCA CATHETER HAVING AN OPTIONALLY FIXATED COREWIRE**
PTCA-KATHETER MIT FAKULTATIV BEFESTIGTEM KERNDRAHT
SONDE POUR ANGIOPLASTIE CORONAIRE TRANSLUMINALE PERCUTANEE DOTEE D'UN FIL CENTRAL EVENTUELLEMENT FIXE

(30) Priority: 03.07.1990 US 547665
(43) Date of publication of application: 21.04.1993
(73) Proprietor: MALLINCKRODT MEDICAL, INC., St. Louis, MO 63134 (US)
(72) Inventor: JENDERSEE, Bradly, A., Missouri City, TX 77459 (US); SOLANO, Scott, J., Lake Jackson, TX 77566 (US)
(74) Representative: Eyles, Christopher Thomas
(86) International application number: US9104753
(87) International publication number: WO9200775

(56) References cited:
- EP-A- 0 213 748
- EP-A- 0 213 750
- EP-A- 0 279 959
- EP-A- 0 371 486
- DE-A- 3 528 876

## Description

### Background of the Invention

The present invention relates to low profile percutaneous transluminal coronary angioplasty (PTCA) catheters. In particular, the present invention relates to PTCA catheters which allows for optional fixation of a corewire for the catheter.

PTCA procedures typically are performed using a guiding catheter which may be percutaneously introduced into the cardiovascular system of the patient through the brachial or femoral arteries and advanced therein until the tip thereof is in the ostium of the desired coronary artery. A guidewire and a dilatation balloon catheter are then introduced through the guiding catheter, the guidewire being disposed within an inner lumen of the balloon catheter. The guidewire and balloon are advanced until the dilatation balloon in properly located within the area of lesion to be treated. Once positioned, the dilatation balloon in inflated to a predetermined size using a radiopaque liquid, such as contrast medium, in order to radially compress the atherosclerotic plaque of the lesion against the inside of the artery wall and thereby dilate the lumen of the artery. The dilatation balloon is then deflated and the balloon catheter removed so that blood flow may be resumed through the dilated artery.

Prior art PTCA catheters have typically employed one of three basic designs: over the wire (OTW), fixed wire (FW) or semi-movable wire (SMW), each of which has its own distinct advantages and disadvantages. The user of such prior art catheters has been forced to accept the disadvantages associated with the chosen design in order to gain the particular advantages provided by the chosen design.

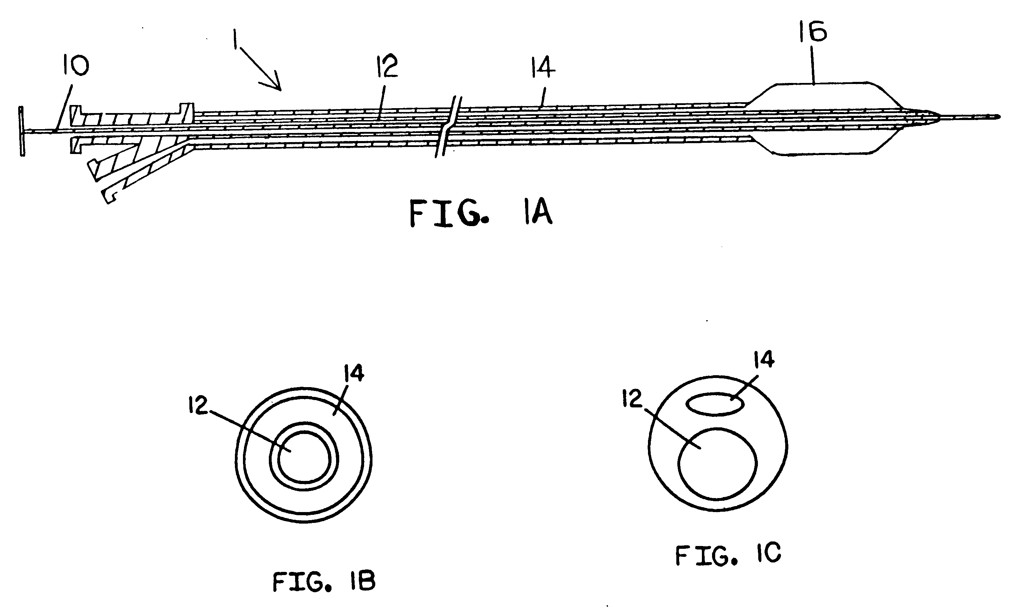

Over the wire (OTW) catheters are the oldest type of PTCA catheter available. FIG. 1A shows a typical design for an OTW catheter, generally designated by reference numeral 1, which employs a two lumen shaft, the two lumens being either coaxially disposed (FIG. 1B) or provided in a multi-lumen configuration (FIG. 1C). A first lumen 12, passes through the entire length of the catheter 1, and accepts an independently manipulated guidewire 10. A second lumen 14, communicates with the inside of a dilatation balloon 16, and constitutes a passageway for injection of contrast medium into the balloon 16 for inflation thereof. In operation of the OTW catheter 1, the guidewire 10, is first passed through the artery to be treated to the area of lesion, and then is passed on through the area of lesion. The catheter 1, is then advanced over the guidewire 10, by slidingly engaging the first lumen 12, over the guidewire 10, until the balloon 16, is properly located within the area of lesion. The balloon 16, is then inflated using contrast media supplied through lumen 14, to radially compress the atherosclerotic plaque in the lesion area against the artery wall. Following dilation of the artery, the catheter 1, and guidewire 10, may be removed. Alternatively, the catheter 1, alone may be removed while leaving the guidewire 10, in place, thus maintaining access to the artery for other devices that may be required.

Advantages of OTW catheters include the ability to remove the catheter while leaving the guidewire in place, thus providing access to the artery for other devices without loss of position within the area of lesion, as noted above. This also provides a relatively high margin of safety, because maintaining access to the artery is crucial should the lesion abruptly reclose following dilation, an occurrence which takes place in about six percent (6%) of all cases. In addition, the OTW catheter has the advantage of allowing the user to choose specific guidewires and catheters independently thereby allowing the user to determine the optimal combination for each case.

A main disadvantage of the OTW catheter is the relatively large profile needed to house the two discrete lumens. This is particularly a problem when it is necessary to cross a tight lesion. Further, the OTW catheter requires relatively complex operation because of the necessity of manipulating two independent elements of the catheter; i.e. the guidewire and the balloon catheter.

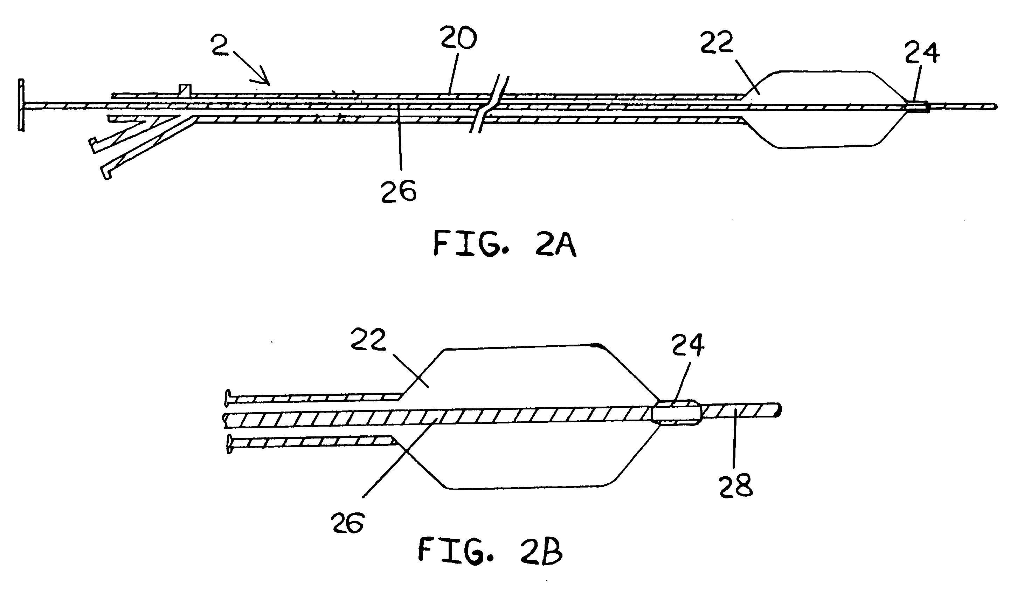

FIGs. 2A and 2B show a typical fixed wire (FW) catheter, generally designated by reference numeral 2, which utilizes a single lumen shaft 20, which communicates directly with the proximal end of a balloon 22, and is sealed at a distal end 24, located distally to the balloon. A tapered corewire 26, similar to that inside a conventional guidewire, runs the entire length of the catheter 2, inside the single lumen, and extends beyond the distal end 24, of the catheter 2, terminating in a conventional guidewire tip 28. The shaft 20, is sealed directly to the core wire 26, at the distal end 24, by suitable sealing means. In operation, the FW catheter 2, sealed to the core wire 26, is guided as a single unit, through the artery to be treated, by manipulating the entire device like a guidewire until the balloon 22, is properly positioned within the area of lesion. Dilatation is then carried out by supplying contrast medium through the single lumen to inflate the balloon 22. Following dilatation, the entire device, i.e. the catheter 2, with sealed core wire 26, is removed.

A major advantage of an FW catheter, is the relatively small profile enabled by the utilization of a single lumen in the catheter shaft, with no lumen present in the area under the balloon. The deflated balloon profile for an FW catheter is consequently considerably smaller than the deflated balloon profile for an OTW catheter. In addition, the shaft diameter of an FW catheter is smaller, which allows for easier and better injections of contrast medium through the guiding catheter and around the shaft of the FW catheter. Also, an FW catheter has the advantage of being relatively easy to use. Because all elements of an FW catheter are fused together, it operates as a single element and may be manipulated easily by a single operator, thus making the dilatation procedure simpler and faster. Another advantage of an FW catheter relates to the pushibility of the catheter. Because the core wire is firmly attached to the distal end of the shaft, the operator can transmit a large amount of longitudinal force to the catheter, aiding in pushing the device across lesions in tortuous areas.

A major disadvantage of an FW catheter is the loss of position within the artery upon removal of the catheter following dilatation. Because an FW catheter acts as a single element, the entire catheter must be removed following dilatation, resulting in a loss of easy access to the artery being treated. Therefore, if it is necessary to insert a further device into the lesion area of the artery, a second wire or catheter must be inserted to recross the lesion, which increases the difficulty and risks of the procedure. A second disadvantage of an FW catheter is a compromise in the steerability of the tip of the catheter. Although the corewire is essentially like a guidewire, the distal end of the catheter shaft is fixed near the end of the corewire, with the result that the balloon tends to act as an anchor on the core wire and thus reduces steerability and performance.

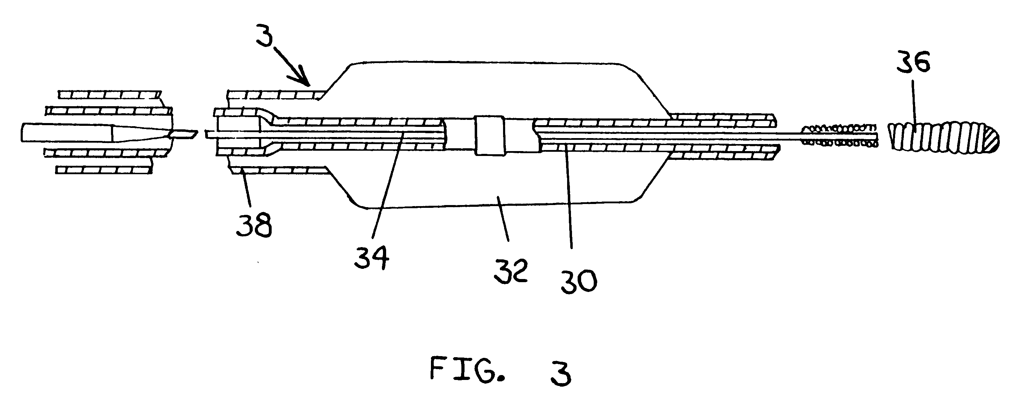

A semi-moveable wire (SMW) catheter is shown in FIG. 3 and represents a compromise between an OTW catheter and an FW catheter. In particular, an SMW catheter, generally designated by reference numeral 3, utilizes a coaxial two lumen design similar to that of an OTW catheter as shown in FIG. 1B, so that a guidewire 34, can move freely within a first lumen 30. A second lumen 38, is provided as means for injecting contrast medium into a balloon 32. The first lumen 30, and second lumen 38, are fused together at their distal ends. In order to decrease profile, the portion of the first lumen 30, under the balloon 32, is tapered to a much smaller size than the portion of the first lumen 30, in the proximal portion of the catheter 3. The guidewire 34, is also tapered so as to fit into the taper of the first lumen 30, while maintaining free rotation and allowing advancement to a small extent (usually about 12 cm). A standard guidewire tip 36, is attached to the end of the guidewire 34. An SMW catheter is operated in much the same manner as an FW catheter. However, because the guidewire 34, may freely rotate and be advanced independently of the catheter shaft, steering of the guidewire 34, across a lesion may be accomplished without the balloon 32, and shaft acting as an anchor. Upon completion of dilatation, the entire SMW catheter, including the guidewire 34, must be removed. This is because the tapered portion of the first lumen 30, is smaller in diameter than the guidewire 34, in the proximal end of the catheter 3, as well as being smaller in diameter than the guidewire tip 36. Therefore, the tapered portion of the inner lumen 30, is bracketed by larger portions of the guidewire 34, and simultaneous removal of both the catheter 3, and guidewire 34, is necessitated.

Advantages of an SMW catheter include a smaller profile and greater simplicity of use as compared to conventional OTW catheters. Also, an SMW catheter has the advantage of better wire movement, i.e. steerability, as compared to an FW catheter.

An SMW catheter has the same disadvantages as an FW catheter as related to maintaining access to the lesion area following dilatation. Further, an SMW catheter has a disadvantage of having a larger profile as compared to FW catheters.

EP-A-0371486 which is considered to represent the closest prior art with respect to the present invention discloses a vascular catheter including an inner tubular member which has a central lumen that extends to the distal tip of the catheter and is adapted to receive a guidewire slidably. The inner tubular member also includes an inflatable collar which, upon inflation, serves to secure or fix the guidewire releasably within its central lumen so that the catheter and guidewire can be advanced through a patient's vasculature as a unit. The catheter also has an inflatable balloon member surrounding the inner tubular member and sealed to it at the distal tip of the catheter.

### Objects of the Present Invention

It is an object of the present invention to provide a single catheter that can be used as an OTW catheter, an FW catheter, and an SMW catheter.

It is a further object of the present invention to provide a catheter that can be operated as any of an OTW, FW, or SMW catheter and which allows the operator to change from one to the other at any time during an angioplasty procedure.

It is a further object of the present invention to provide a catheter that contains an inflatable seal at its distal end, for the purpose of selectively fixing a corewire within the tip of the catheter.

It is a further object of the present invention to provide a catheter that contains an inflatable seal at its distal end, for the purpose of purging air from the catheter prior to use.

### Summary of the Invention

The objects of the present invention are accomplished by providing a PTCA catheter comprising a multi-lumen shaft having a first large main lumen through which a corewire may be provided and through which contrast medium may be supplied in order to inflate a balloon attached near the distal end of the catheter shaft. The multi-lumen shaft also includes a second small lumen which is in axially fixed relationship with the first large main lumen and travels the entire length of the catheter, terminating at a small balloon which acts as an inflatable seal, disposed within the distal tip of the catheter shaft. By inflating the inflatable seal, a corewire passing through the first lumen may be locked in place so that the catheter acts as an FW catheter. Once inflated, the inflatable seal also seals the distal end of the catheter, allowing the dilatation balloon to be inflated. When the inflatable seal is in a deflated condition, free rotation and longitudinal movement of the corewire is enabled so that the catheter acts as an SWM catheter. Moreover, relative sizes of the corewire and first lumen are such that the catheter shaft may be entirely removed from the corewire while leaving the corewire in place within the artery being treated, thus providing all of the advantages of an OTW catheter. The inflatable seal in the tip of the catheter according to the present invention, may be inflated and deflated repeatedly and at any time during a dilatation procedure in order to give the operator the option of using the optimal type of device (i.e. OTW, FW or SMW catheter) at different stages of the procedure.

### Brief Description of the Drawings

FIG. 1A is a plan view of a typical OTW catheter as known in the prior art.

FIG. 1B is a cut away end view of an OTW catheter shaft having coaxially disposed lumens as known in the prior art.

FIG. 1C is a cut away end view of an OTW catheter shaft having a multi-lumen configuration as known in the prior art.

FIG. 2A is a plan view of a typical FW catheter as known in the prior art.

FIG. 2B is a expanded view of the distal portion of the FW catheter shown in FIG. 2A.

FIG. 3 is a plan view of a typical SMW catheter as known in the prior art.

FIG. 4A is a plan view of a catheter according to the present invention.

FIG. 4B is a cross-sectional end view of the catheter shaft according to the present invention taken along line B-B of FIG. 4A.

FIG. 4C is a cross-sectional end view of the catheter shaft according to the present invention taken along line C-C of FIG. 4A.

FIG. 4D is a cross-sectional end view of the catheter shaft according to the present invention taken along line D-D of FIG. 4A.

### Detailed Description of the Present Invention

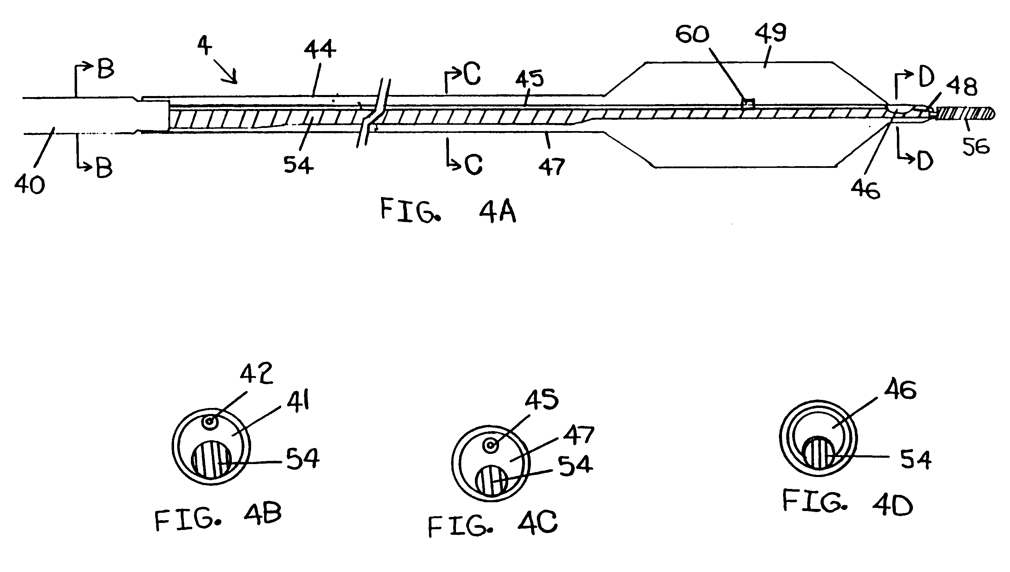

FIG. 4A is a plan view of a catheter, generally designated by reference numeral 4, according to the present invention. In particular, the catheter 4, includes a multi-lumen proximal shaft 40, having a large main lumen 41, and a very small secondary lumen 42. (See FIG. 4B). The proximal shaft 40, is bonded to a balloon extension 44, proximal to a dilatation balloon 49. The main lumen 41, communicates directly with the interior of an outer member 47, of the balloon extension 44, while the secondary lumen 42, is bonded to a small inner member 45, of the balloon extension 44. (See FIG. 4C). The inner member 45, extends the entire length of the balloon extension 44, and terminates at a small inflatable seal 46, disposed within a distal tip 48, of the catheter 4. (See FIG. 4D). The outer member 47, also extends the entire length of the balloon extension 44, including a section making up the dilatation balloon 49, and ultimately opens at the distal tip 48, of the catheter 4. A single radiopaque marker band 60, is positioned on the inner member 45, to fluoroscopically delineate the center of the dilatation balloon 49.

The catheter 4, terminates at its proximal end in a standard adjustable Y connector (not shown). The main lumen 41, continues through the length of the connector, and terminates at a gasket (not shown). The gasket, allows a corewire 54, to pass therethrough while providing a seal around the corewire 54, which prevents fluid from leaking out the proximal end of the catheter 4. The corewire 54, passes through the entire length of the main lumen 41, and the outer member 47, and emerges from the opening in the distal tip 48, of the catheter 4. The connector includes a first inflation leg, communicating with the secondary lumen 42, and allowing for independent inflation of the inflatable seal 46. The connector further includes a second inflation leg communicating with the main lumen 41, and allowing for inflation of the dilatation balloon 49.

Preferably, all of the components of the catheter 4, are composed of oriented polyethylene so that the components may be heat bonded together, thus simplifying manufacturing carried out using well known processes. The proximal shaft 40, may be formed of a high density polyethylene material, which provides a relatively stiff shaft having increased pushibility and a low friction coefficient for better corewire movement. The inner member 45, and the outer member 47, of the balloon extension 44, may be formed of a linear low density polyethylene to provide flexibility and strength. The outer member 47, may also be formed of other materials that provide various combinations of pushibility and flexibility, such as, PET, Nylon, Nylon blends, stainless steel, polyimide, etc. The inner member 45, is preferably formed of oriented linear low density polyethylene, so that it may be heat bonded to the other components of the catheter. Other materials could be used as well, including PET, Nylon, Nylon blends, polyimide, or elastomeric materials such as silicone or latex.

The inflatable seal 46, is preferably formed as an integral portion of the inner member 45, by heating the end portion or the inner member 45, while expanding the end portion under pressure. The dilatation balloon 49, may be formed as an integral portion of the outer member 47, in the same manner. By forming the balloons as integral portions of the members, the need for separately bonding balloons onto the members is obviated. The inflatable seal 46, may be heat bonded to the interior surface of the outer member 47, by deflating the inflatable seal 46, and inserting a mandrel inside the lumen of the inner member 45, to provide support. The integrities of the inflatable seal 46, and dilatation balloon 48, can be maintained by controlling the application of heat during formation. The inflatable seal 46, may also be made of an elastomeric material, such as silicone, latex rubber, or polyurethane and adhesively bonded to the inner member 45, and distal tip 48.

The proximal end of the balloon extension 44, is preferably heat bonded to the distal end of the proximal shaft 40, in a well known manner. Further, the proximal shaft 40, and balloon extension 44, are preferably bonded at a position 5 to 35 cm proximal to the dilatation balloon 49. The outer diameter of the main lumen 41, and outer member 47, are approximately the same in order to assure a smooth transition and strong bond therebetween. Also, the inner diameter of the secondary lumen 42, and the inner member 45, are approximately the same to assure a smooth transition and strong bond therebetween. The diameter of the main lumen 41, and outer member 47, is preferably in the range of 3 to 6 times greater than the diameter of the secondary lumen 42, and inner member 45. The relatively small diameter of the secondary lumen 42, and inner member 45, allows the catheter 4, to approximate the overall dimensions of a single lumen catheter, thus reducing the profile to that of a FW catheter, while maintaining the advantages of an OTW catheter.

The corewire 54, is preferably of a tapered configuration, tailored to provide the optimal combination of flexibility and pushibility. The diameter of the proximal untapered portion of the corewire 54, is preferably in a range from .010" to .020" (0.254 to 0.508 mm). The taper preferably begins about 15 to 35 cm from the distal tip of the corewire 54, and includes at least two discrete tapers separated by a length of untapered core. Most preferably, the portion of the corewire 54, which will be in place within the dilatation balloon 49, has a diameter of about .006" (0.152 mm) and then quickly tapers toward the distal end of the corewire 54, ending in a coil of radiopaque metal 56. The small diameter of the corewire 54, under the dilatation balloon 49, helps to minimize the profile of the deflated dilatation balloon 49. The corewire 54, may be formed of any suitable material, but is preferably formed of stainless steel or an alloy of nickel and titanium (nitinol).

It is also preferable to coat the surface of the corewire 54, with a lubricous material, such as, Teflon, silicone, hydrogel or other substances, in order to reduce friction between the corewire 54, and the outer member 47. In addition, the outer surface of the catheter 4, may be coated with a similar lubricous substance, such as silicone or hydrogel, in order to reduce friction of the outer surface of the catheter 4, during use.

The catheter according to the present invention may operate as any one of an OTW, FW, or SMW catheter at the option of the physician. Further, the physician may select the type of catheter he would like to use, at any time during the angioplasty procedure. This selectivity is enabled by the inclusion of the inflatable seal in the distal tip of the catheter. Further advantages of the catheter according to the present invention, include the ability to rapidly purge air from the catheter, and the relatively low profile of the catheter shaft and dilatation balloon made possible by the elimination of a large inflation lumen.

The overall design of the catheter according to the present invention is similar to an FW catheter, in that a single lumen is utilized for both inflation/deflation of the dilatation balloon and for passage of the corewire. This single lumen design assures a small profile for the catheter shaft and dilatation balloon. However, the catheter according to the present invention can also act as an SMW catheter, because the inflatable seal in the distal tip of the catheter allows the corewire to move freely within the main lumen. In other words, unlike a standard FW catheter, the corewire in the catheter according to the present invention does not have to be sealed at the distal tip of the catheter, thereby allowing the free movement of the corewire. Upon inflation of the inflatable seal the corewire is locked into place so that the catheter may operate as an FW catheter. Further, inflation of the inflatable seal seals the distal tip of the catheter, so that inflation and deflation of the dilatation balloon may be carried out through the main lumen. In addition, the catheter according to the present invention provides all of the advantages of an OTW catheter. In particular, if a corewire is already in place within the patient, the corewire supplied with the present catheter nay be removed, and then the catheter shaft may be introduced to the patient over the corewire already in place. Also, if it is desired to introduce further devices following dilatation using the present catheter, the catheter shaft may be removed while leaving the corewire in place, thus maintaining access to the treatment area.

One method of using the catheter according to the present invention is described below.

As with all balloon catheters, air within the catheter must be purged from the catheter shaft and dilatation balloon prior to introduction of the catheter into the patient. This purging is necessitated by the need for an air-free hydraulic system to enhance inflation pressure, and also by the need to protect against injection of air into the coronary artery should the balloon rupture during the angioplasty procedure. In the catheter according to the present invention, this purging may be performed very easily and quickly, thus providing one advantage of the present invention. By drawing a vacuum on the inflatable seal the distal tip of the main lumen is entirely open, so that purging of air may be easily accomplished by injecting contrast medium through the main lumen from the proximal end and out the distal tip, until all air is seen to exit from the distal tip. This purging may be accomplished very rapidly when compared to prior art catheters because the distal tip opening of the catheter according to the present invention is much larger than the small pores or opening provided for purging purposes in the prior art catheters.

The catheter according to the present invention will normally be packaged with the corewire already installed through the main lumen. However, if the physician already has a corewire in place within the patient, the corewire may be removed and the catheter may be used as an OTW catheter. In other words, the catheter may be introduced to the patient over the corewire already in place. Once introduced, the catheter may be locked into place over the existing corewire, at any time, by simply inflating the inflatable seal in the distal tip of the catheter. Therefore, the enhanced pushibility of an FW catheter may by attained. In any case, once the balloon is properly located within the area of treatment, the inflatable seal is inflated to seal the distal end of the catheter, and inflation/ deflation of the dilatation balloon may be carried out by injecting contrast medium through the main lumen and into the dilatation balloon. Once an initial dilation has been carried out, the physician continues to have several options. In particular, if it desired to introduce a further device to the treatment area, the catheter may be removed while leaving the corewire in place. Further, the catheter may be used as any of an OTW, FW, or SMW catheter to access and dilate other lesions as the physician feels appropriate.

In one alternative, if the physician initially prefers an FW catheter, the inflatable seal may be inflated immediately following purging, in order to lock the corewire in place. The corewire and catheter may then be guided to the area of treatment within the artery as a single unit. When the dilatation balloon has been properly located within the lesion, the dilatation balloon may be immediately inflated as described above. Following this initial dilation, the physician has the same options as described above, (i.e. leave the corewire in place or continue to use the catheter as any one of an OTW, FW, or SMW catheter).

In a further, alternative, if the physician initially prefers an SMW catheter, the inflatable seal is not inflated, thus allowing the corewire to move freely. The corewire may then be manipulated through the area of lesion, followed by proper positioning of the dilatation balloon over the corewire. Once the dilatation balloon is in the proper position, the inflatable seal is inflated to seal the distal tip of the catheter, and then inflation/deflation of the dilatation balloon is carried out as described above. Again, once the dilation is complete, the physician has all of the options noted above.

It should be further noted, that the catheter according to the present invention, may act as any of an OTW, FW, or SMW catheter at any time during the procedure, by simply inflating or deflating the inflatable seal. For example, the physician has the option of beginning the procedure with the inflatable seal inflated and the corewire locked in place and acting as an FW catheter, and then at a later stage of the procedure, deflating the inflatable seal thus releasing the corewire, so that the catheter acts as a SMW catheter. An infinite number of combinations and changes from one type of catheter to another may be carried out during a single angioplasty procedure, at the option of the physician. Therefore, the catheter according to the present invention is highly advantageous in providing all of the advantages of each type of catheter along with the versatility and ease of switching between the different types of catheters at any time during the angioplasty procedure.

The foregoing has been a description of certain preferred embodiments of the present invention, but is not intended to limit the invention in any way. Rather, many modifications, variations and changes in details may be made within the scope of the present invention.

## Claims

1. An angioplasty catheter comprising:
a catheter shaft (40) having a proximal end and a distal end and having a main lumen (41) and a secondary lumen (42) extending in axially fixed relationship therethrough from the proximal end to the distal end;
a balloon extension (44) having a proximal end and a distal end, the proximal end of said balloon extension being connected to the distal end of said catheter shaft (40), said balloon extension (44) having an outer member (47) and an inner member (45) extending from the proximal end to the distal end of the balloon extension (44);
the proximal end of said outer member (47) being continuous with said main lumen (41) at its distal end; the distal end of said outer member (47) comprising a distal tip (48) of said catheter; and said outer member (47) having a dilation balloon (49) formed in a portion of its length; and
said inner member (45) being connected at its proximal end to the secondary lumen (42) at its distal end;
characterised in that the outer member (47) opens at the distal tip (48);
that the distal end of the inner member (45) terminates in an inflatable seal (46) disposed within said distal tip (48) of said catheter; and
that the inflatable seal (46) is arranged in its inflated state to seal the distal tip (48) of the catheter to enable inflation and deflation of the dilatation balloon (49) to be carried out through the main lumen (41).

2. An angioplasty catheter according to claim 1, characterised in that the catheter shaft is a coaxial two lumen catheter shaft, having an outer member extending therethrough from the proximal end to the distal end, and an inner member coaxially located within said outer member and also extending therethrough from the proximal end to the distal end.

3. An angioplasty catheter according to claim 1 or claim 2, characterised in that the diameter of said main lumen (41) is 3 to 6 times the diameter of said inner member (45).

4. An angioplasty catheter according to any one of claims 1 to 3, characterised in that the outer diameter of said main lumen (41) is approximately the same as the outer diameter of said outer member (44), and the diameter of said secondary lumen (42) is approximately the same as the diameter of the inner member (45).

5. An angioplasty catheter according to any one of claims 1 to 4, characterised in that at least one of said inner member (45) and said outer member (44) is formed of oriented polyethylene.

6. An angioplasty catheter according to any one of claims 1 to 5, characterised in that said outer member (44) is formed from high density polyethylene.

7. An angioplasty catheter according to any one of claims 1 to 6, characterised in that at least one of said inner member (45) and said outer member (44) is formed from linear low density polyethylene.

8. An angioplasty catheter according to any one of claims 1 to 7, characterised in that said dilatation balloon (49) is formed as a continuous part of said outer member (44), by heating a portion of said outer member and applying pressure to inflate that portion.

9. An angioplasty catheter according to any one of claims 1 to 8, characterised in that said dilatation balloon (49) is formed as a balloon extension member, and said balloon extension member is heat bonded to said outer member (44).

10. An angioplasty catheter according to claim 9, characterised in that said dilatation balloon (49) is formed from linear low density polyethylene and is bonded to said outer member (44).

11. An angioplasty catheter according to any one of claims 1 to 10, characterised in that said catheter shaft (40) and said balloon extension (47) are formed from oriented polyethylene.

12. An angioplasty catheter according to claim 1 to 11, characterised in that said catheter shaft (40) is heat bonded to said balloon extension (47).

13. An angioplasty catheter according to claim 1 to 12, characterised in that said catheter shaft (40) is formed from high density polyethylene.

14. An angioplasty catheter according to any one of claims 1 to 13, characterised in that said balloon extension (49) is formed from linear low density polyethylene.

15. An angioplasty catheter according to any one of claims 1 to 14, characterised in that said inflatable seal (46) is formed from said inner member (45) by heating a portion of said inner member and applying pressure to inflate said inner member, thus forming a small balloon segment continuous with said inner member.

16. An angioplasty catheter according to any one of claims 1 to 14, characterised in that said inflatable seal (46) is formed from an elastomeric material such as silicone, latex, or polyurethane and is bonded to the proximal end of said inner member (45) and in a position distal to said distal tip (48) of said catheter.

17. An angioplasty catheter according to claim 15 or claim 16, characterised in that said inflatable seal (46) is heat bonded within said distal tip (48) of said catheter.

18. An angioplasty catheter according to any one of claims 1 to 17m characterised in that it further includes a tapered corewire (54) extending through the entire length of said main lumen (41) and said outer member (44) and extending out said distal tip (48) of said catheter, the distal end of said tapered corewire terminating with a coil of radiopaque metal (56).

19. An angioplasty catheter according to claim 18, characterised in that said tapered corewire (54) is made of stainless steel, or of an alloy of nickel and titanium (nitinol).

20. An angioplasty catheter according to claim 18 or claim 19, characterised in that said tapered corewire (54) has a diameter of about .006" (about 0.152 mm) along a portion extending through said dilatation balloon.

21. An angioplasty catheter according to any one of claims 18 to 20, characterised in that said tapered corewire (54) is coated with Teflon, silicone, hydrogel, or other lubricous substance to reduce friction within said outer member.

22. An angioplasty catheter according to any one of claims 1 to 21, characterised in that said catheter shaft (40) and said balloon extension (47) are bonded together at a position 5 to 35 cm proximal to the dilatation balloon (49).

23. An angioplasty catheter according to any one of claims 1 to 22, characterised in that it further includes first means for independently inflating and deflating said inflatable seal (46) and second means for independently inflating and deflating said dilation balloon (49).

24. An angioplasty catheter according to any one of claims 1 to 23, characterised in that the outer surface of said catheter is coated with a lubricous substance such as silicone or hydrogel, to reduce friction on the outer surface of said catheter.

25. A method for purging an angioplasty catheter comprising the steps of: deflating s selectively inflatable seal (46) located in the distal end of said catheter; allowing free efflux of air and fluid from an inflation lumen (42) and dilatation balloon (49) of said catheter; and inflating said inflatable seal to prevent leakage from said distal end (48) of said catheter during use.

## Patentansprüche

1. Angioplastie-Katheter mit
einem Katheterschaft (40) mit einem proximalen Ende und einem distalen Ende und einem Hauptlumen (41) und einem sekundären Lumen (42), die axial fixiert von dem proximalen Ende zu dem distalen Ende durch ihn hindurchgehen,
einer Ballonverlängerung (44) mit einem an das distale Ende des Katheterschaftes (40) angeschlossenen proximalen Ende und einem distalen Ende, sowie mit einem äußeren Element (47) und einem inneren Element (45), die sich von dem proximalen Ende zu dem distalen Ende der Ballonverlängerung (44) erstrecken,
wobei das proximale Ende des äußeren Elements (47) an das distale Ende des genannten Hauptlumens (41) angeschlossen ist, das distale Ende des äußeren Elements (47) eine distale Spitze (48) des Katheters aufweist und das äußere Element (47) einen in einem Teil seiner Länge ausgebildeten Erweiterungsballon (49) hat und
das innere Element (45) an seinem proximalen Ende an das distale Ende des sekundären Lumens (42) angeschlossen ist,
dadurch gekennzeichnet, daß das äußere Element (47) an der distalen Spitze (48) offen ist,
daß das distale Ende des inneren Elements (45) in einer innerhalb der distalen Spitze (48) des Katheters angeordneten, aufblasbaren Dichtung (46) endet, und
daß die aufblasbare Dichtung (46) in ihrem aufgeblasenen Zustand so ausgebildet ist, daß die distale Spitze (48) des Katheters abgedichtet wird, so daß der Erweiterungsballon (49) durch das Hauptlumen (41) aufgeblasen und abgeblasen werden kann.

2. Angioplastie-Katheter nach Anspruch 1, dadurch gekennzeichnet, daß der Katheterschaft ein koaxialer Doppellumen-Katheterschaft ist mit einem von dem proximalen Ende zu dem distalen Ende hindurchgehenden äußeren Element und einem in dem äußeren Element koaxial angeordneten und von dem proximalen Ende zu dem distalen Ende ebenfalls hindurchgehenden inneren Element.

3. Angioplastie-Katheter nach Anspruch 1 oder Anspruch 2, dadurch gekennzeichnet, daß der Durchmesser des Hauptlumens (41) gleich dem 3- bis 6-fachen Durchmesser des genannten inneren Elements (45) ist.

4. Angioplastie-Katheter nach einem der Ansprüche 1 bis 3, dadurch gekennzeichnet, daß der Außendurchmesser des Hauptlumens (41) etwa gleich dem Außendurchmesser des äußeren Elements (44) ist und der Durchmesser des sekundären Lumens (42) etwa gleich dem Durchmesser des inneren Elements (45) ist.

5. Angioplastie-Katheter nach einem der Ansprüche 1 bis 4, dadurch gekennzeichnet, daß das innere Element (45) und/oder das äußere Element (44) aus orientiertem Polyethylen gebildet ist.

6. Angioplastie-Katheter nach einem der Ansprüche 1 bis 5, dadurch gekennzeichnet, daß das äußere Element (44) aus hochdichtem Polyethylen gebildet ist.

7. Angioplastie-Katheter nach einem der Ansprüche 1 bis 6, dadurch gekennzeichnet, daß das innere Element (45) und/oder das äußere Element (44) aus linearem Polyethylen niedriger Dichte gebildet ist.

8. Angioplastie-Katheter nach einem der Ansprüche 1 bis 7, dadurch gekennzeichnet, daß der Erweiterungsballon (49) als kontinuierlicher Teil des äußeren Elements (44) durch Erhitzen eines Teils des äußeren Elements und Druckanwendung zum Aufblasen des Teils gebildet ist.

9. Angioplastie-Katheter nach einem der Ansprüche 1 bis 8, dadurch gekennzeichnet, daß der Erweiterungsballon (49) als ein Ballonverlängerungselement ausgebildet ist und das Ballonverlängerungselement durch Heißbindung mit dem äußeren Element (44) verbunden ist.

10. Angioplastie-Katheter nach Anspruch 9, dadurch gekennzeichnet, daß der Erweiterungsballon (49) aus linearem Polyethylen niedriger Dichte gebildet ist und an das äußere Element (44) gebunden ist.

11. Angioplastie-Katheter nach einem der Ansprüche 1 bis 10, dadurch gekennzeichnet, daß der Katheterschaft (40) und die Ballonverlängerung (47) aus orientiertem Polyethylen gebildet sind.

12. Angioplastie-Katheter nach Anspruch 1 bis 11, dadurch gekennzeichnet, daß der Katheterschaft (40) durch Heißbindung mit der genannten Ballonverlängerung (47) verbunden ist.

13. Angioplastie-Katheter nach Anspruch 1 bis 12, dadurch gekennzeichnet, daß der Katheterschaft (40) aus Polyethylen hoher Dichte gebildet ist.

14. Angioplastie-Katheter nach einem der Ansprüche 1 bis 13, dadurch gekennzeichnet, daß die Ballonerweiterung (49) aus linearem Polyethylen niedriger Dichte gebildet ist.

15. Angioplastie-Katheter nach einem der Ansprüche 1 bis 14, dadurch gekennzeichnet, daß die aufblasbare Dichtung (46) aus dem genannten inneren Element (45) durch Erhitzen eines Teils des inneren Elements und Druckanwendung zum Aufblasen des inneren Elements gebildet ist, so daß ein mit dem inneren Element zusammenhängendes, kleines Ballonsegment gebildet wird.

16. Angioplastie-Katheter nach einem der Ansprüche 1 bis 14, dadurch gekennzeichnet, daß die aufblasbare Dichtung (46) aus einem elastomeren Material, wie Silikon, Latex oder Polyurethan, gebildet ist und in einer zu der distalen Katheterspitze (48) distalen Position an das proximale Ende des inneren Elements (45) gebunden ist.

17. Angioplastie-Katheter nach Anspruch 15 oder Anspruch 16, dadurch gekennzeichnet, daß die aufblasbare Dichtung (46) durch Heißbindung in der distalen Spitze (48) des Katheters angebracht ist.

18. Angioplastie-Katheter nach einem der Ansprüche 1 bis 17, dadurch gekennzeichnet, daß er ferner einen sich verjüngenden Kerndraht (54) enthält, der sich durch die Gesamtlänge des Hauptlumens (41) und des äußeren Elements (44) und aus der distalen Spitze (48) des Katheters heraus erstreckt, wobei das distale Ende des sich verjüngenden Kerndrahts in einer Wendel aus radiopakem Metall (56) endet.

19. Angioplastie-Katheter nach Anspruch 18, dadurch gekennzeichnet, daß der sich verjüngende Kerndraht (54) aus Edelstahl oder aus einer Legierung aus Nickel und Titan (Nitinol) hergestellt ist.

20. Angioplastie-Katheter nach Anspruch 18 oder Anspruch 19, dadurch gekennzeichnet, daß der sich verjüngende Kerndraht (54) längs eines sich durch den Erweiterungsballon erstreckenden Teils einen Durchmesser von etwa 0,006" (etwa 0,152 mm) hat.

21. Angioplastie-Katheter nach einem der Ansprüche 18 bis 20, dadurch gekennzeichnet, daß der sich verjüngende Kerndraht (54) zur Verringerung der Reibung in dem äußeren Element mit Teflon, Silikon, Hydrogel oder einer anderen schlüpfrigen Substanz beschichtet ist.

22. Angioplastie-Katheter nach einem der Ansprüche 1 bis 21, dadurch gekennzeichnet, daß der Katheterschaft (40) und die Ballonverlängerung (47) an einer Stelle miteinander verbunden sind, die einen Abstand von 5 bis 35 cm bis zum Erweiterungsballon (49) hat.

23. Angioplastie-Katheter nach einem der Ansprüche 1 bis 22, dadurch gekennzeichnet, daß er ferner erste Einrichtungen zum unabhängigen Auf- und Abblasen der aufblasbaren Dichtung (46) und zweite Einrichtungen zum unabhängigen Auf- und Abblasen des Erweiterungsballons (49) umfaßt.

24. Angioplastie-Katheter nach einem der Ansprüche 1 bis 23, dadurch gekennzeichnet, daß die äußere Oberfläche des Katheters mit einer schlüpfrigen Substanz, wie Silikon oder Hydrogel, beschichtet ist, um die Reibung auf der Außenseite des Katheters zu verringern.

25. Verfahren zur Entleerung eines Angioplastie-Katheters, bei dem man eine an dem distalen Katheterende befindliche, selektiv aufblasbare Dichtung (46) abbläst, aus einem Aufblaslumen (42) und Erweiterungsballon (49) des Katheters Luft und Strömungsmittel frei ausfließen läßt und die aufblasbare Dichtung aufbläst, um ein Ausströmen aus dem distalen Ende (48) des Katheters während der Benutzung zu verhindern.

## Revendications

1. Cathéter pour angioplastie comprenant :
une tige de cathéter (40) ayant une extrémité proximale et une extrémité distale et ayant une lumière principale (41) et une lumière secondaire (42) qui s'étendent dans celle-ci de l'extrémité proximale à l'extrémité distale selon une relation axialement fixée ;
un prolongement à ballonnet (44) ayant une extrémité proximale et une extrémité distale, l'extrémité proximale dudit prolongement à ballonnet étant reliée à l'extrémité distale de ladite tige de cathéter (40), ledit prolongement à ballonnet (44) ayant un élément externe (47) et un élément interne (45) qui s'étendent de l'extrémité proximale à l'extrémité distale du prolongement à ballonnet (44) ;
l'extrémité proximale dudit élément externe (47) étant continue avec ladite lumière principale (41) à son extrémité distale, l'extrémité distale dudit élément externe (47) comprenant un embout distal (48) dudit cathéter, et ledit élément externe (47) ayant un ballonnet de dilatation (49) formé dans une partie de sa longueur ; et
ledit élément interne (45) étant relié à son extrémité proximale à la lumière secondaire (42) à son extrémité distale ;
caractérisé en ce que l'élément externe (47) s'ouvre au niveau de l'embout distal (48) ;
en ce que l'extrémité distale de l'élément interne (45) se termine par un joint gonflable (46) disposé à l'intérieur dudit embout distal (48) dudit cathéter ; et
en ce que le joint gonflable (46) est agencé dans son état gonflé pour fermer hermétiquement l'embout distal (48) du cathéter pour permettre au gonflage et au dégonflage du ballonnet de dilatation (49) d'être réalisés par l'intermédiaire de la lumière principale (41).

2. Cathéter pour angioplastie selon la revendication 1, caractérisé en ce que la tige de cathéter est une tige de cathéter à deux lumières coaxiales ayant un élément externe qui s'étend suivit celle-ci de l'extrémité proximale à l'extrémité distale et un élément interne situé coaxialement à l'intérieur dudit élément externe et qui s'étend également suivant celle-ci de l'extrémité proximale à l'extrémité distale.

3. Cathéter pour angioplastie selon la revendication 1 ou la revendication 2, caractérisé en ce que le diamètre de ladite lumière principale (41) est 3 à 6 fois le diamètre dudit élément interne (45).

4. Cathéter pour angioplastie selon l'une quelconque des revendications 1 à 3, caractérise en ce que le diamètre externe de ladite lumière principale (41) est approximativement identique au diamètre externe dudit élément externe (44), et le diamètre de ladite lumière secondaire (42) est approximativement identique au diamètre de l'élément interne (45).

5. Cathéter pour angioplastie selon l'une quelconque des revendications 1 à 4, caractérisé en ce qu'au moins un élément parmi ledit élément interne (45) et ledit élément externe (44) est constitué par du polyéthylène orienté.

6. Cathéter pour angioplastie selon l'une quelconque des revendications 1 à 5, caractérisé en ce que ledit élément externe (44) est constitué par du polyéthylène haute densité.

7. Cathéter pour angioplastie selon l'une quelconque des revendications 1 à 6, caractérisé en ce qu'au moins un élément parmi ledit élément interne (45) et ledit élément externe (44) est constitué par du polyéthylène linéaire basse densité.

8. Cathéter pour angioplastie selon l'une quelconque des revendications 1 à 7, caractérisé en ce que ledit ballonnet de dilatation (49) est formé sous forme d'une partie continue dudit élément externe (44) par chauffage d'une partie dudit élément externe et application d'une pression pour gonfler cette partie.

9. Cathéter pour angioplastie selon l'une quelconque des revendications 1 à 8, caractérisé en ce que ledit ballonnet de dilatation (49) est formé sous forme d'un élément de prolongement à ballonnet et ledit élément de prolongement à ballonnet est lié thermiquement audit élément externe (44).

10. Cathéter pour angioplastie selon la revendication 9, caractérisé en ce que ledit ballonnet de dilatation (49) est constitué par du polyéthylène linéaire basse densité et est lié audit élément externe (44).

11. Cathéter pour angioplastie selon l'une quelconque des revendications 1 à 10, caractérisé en ce que ladite tige de cathéter (40) et ledit prolongement à ballonnet (47) sont constitués par du polyéthylène orienté.

12. Cathéter pour angioplastie selon les revendications 1 à 11, caractérisé en ce que ladite tige de cathéter (40) est liée thermiquement audit prolongement à ballonnet (47).

13. Cathéter pour angioplastie selon les revendications 1 à 12, caractérisé en ce que ladite tige de cathéter (40) est constituée par du polyéthylène haute densité.

14. Cathéter pour angioplastie selon l'une quelconque des revendications 1 à 13, caractérisé en ce que ledit prolongement à ballonnet (49) est constitué par du polyéthylène linéaire basse densité.

15. Cathéter pour angioplastie selon l'une quelconque des revendications 1 à 14, caractérisé en ce que ledit joint gonflable (46) est formé à partir dudit élément interne (45) par chauffage d'une partie dudit élément interne et application d'une pression pour gonfler ledit élément interne, pour former ainsi un petit segment de ballonnet continu avec ledit élément interne.

16. Cathéter pour angioplastie selon l'une quelconque des revendications 1 à 14, caractérisé en ce que ledit joint gonflable (46) est constitué par une matière élastomérique telle que la silicone, le latex ou le polyuréthane et est lié à l'extrémité proximale dudit élément interne (45) et dans une position distale audit embout distal (48) dudit cathéter.

17. Cathéter pour angioplastie selon la revendication 15 ou la revendication 16, caractérisé en ce que ledit joint gonflable (46) est lié thermiquement à l'intérieur dudit embout distal (48) dudit cathéter.

18. Cathéter pour angioplastie selon l'une quelconque des revendications 1 à 17, caractérisé en ce qu'il comprend en outre une âme effilée (54) qui s'étend sur toute la longueur de ladite lumière principale (41) et dudit élément externe (44) et qui s'étend à l'extérieur dudit embout distal (48) dudit cathéter, l'extrémité distale de ladite âme effilée se terminant par une bobine de métal radio-opaque (56).

19. Cathéter pour angioplastie selon la revendication 18, caractérisé en ce que ladite âme effilée (54) est constituée par de l'acier inoxydable ou par un alliage de nickel et de titane (nitinol).

20. Cathéter pour angioplastie selon la revendication 18 ou la revendication 19, caractérisé en ce que ladite âme effilée (54) a un diamètre d'environ 0,006" (environ 0,152 mm) sur une partie qui s'étend dans ledit ballonnet de dilatation.

21. Cathéter pour angioplastie selon l'une quelconque des revendications 18 à 20, caractérisé en ce que ladite âme effilée (54) est revêtue de Teflon, de silicone, d'hydrogel ou d'une autre substance lubrifiante pour réduire le frottement à l'intérieur dudit élément externe.

22. Cathéter pour angioplastie selon l'une quelconque des revendications 1 à 21, caractérisé en ce que ladite tige de cathéter (40) et ledit prolongement à ballonnet (47) sont liés entre eux en une position proximale de 5 à 35 cm du ballonnet de dilatation (49).

23. Cathéter pour angioplastie selon l'une quelconque des revendications 1 à 22, caractérisé en ce qu'il comprend en outre des premiers moyens pour gonfler et dégonfler indépendamment ledit joint gonflable (46) et des seconds moyens pour gonfler et dégonfler indépendamment ledit ballonnet de dilatation (49).

24. Cathéter pour angioplastie selon l'une quelconque des revendications 1 à 23, caractérisé en ce que la surface externe dudit cathéter est recouverte d'une substance lubrifiante telle qu'une silicone ou un hydrogel pour réduire le frottement sur la surface externe dudit cathéter.

25. Procédé pour purger un cathéter pour angioplastie comprenant les étapes consistant à dégonfler un joint sélectivement gonflable (46) situé dans l'extrémité distale dudit cathéter, à permettre un écoulement libre d'air et de fluide depuis une lumière de gonflage (42) et un ballonnet de dilatation (49) dudit cathéter, et à gonfler ledit joint gonflable pour empêcher des fuites depuis ladite extrémité distale (48) dudit cathéter pendant l'utilisation.
